# EUROPÄISCHE PATENTSCHRIFT

(11) **EP 0 494 644 B1**
(45) Veröffentlichungstag und Bekanntmachung des Hinweises auf die Patenterteilung: **19.11.1997**
(21) Anmeldenummer: 92100157.4
(22) Anmeldetag: 07.01.1992
(51) Int. Cl.: A61B 17/06

(54) **Chirurgische Nadel**
Surgical needle
Aiguille chirurgicale

(30) Priorität: 07.01.1991 DE 9100109 U
(43) Veröffentlichungstag der Anmeldung: 15.07.1992
(73) Patentinhaber: Scarfi, Andrea, Dr. med., D-78464 Konstanz (DE); SERAG-WIESSNER GmbH & CO. KG, D-95112 Naila (DE)
(72) Erfinder: Scarfi, Andrea, Dr. Med., W-7750 Konstanz (DE)
(74) Vertreter: Klingseisen, Franz, Dipl.-Ing.

(56) Entgegenhaltungen:
- EP-A- 0 404 018
- DE-A- 2 011 838
- DE-A- 3 712 163
- DE-B- 1 283 433
- FR-A- 403 111
- US-A- 2 092 929
- US-A- 2 811 157
- US-A- 4 224 947

## Beschreibung

Die Erfindung betrifft eine chirurgische Nadel nach dem Obergbegriff des Anspruchs 1 und des Anspruchs 2.

Aus US-A-2 811 157 sowie DE-A-2 011 838 ist jeweils eine chirurgische Nadel bekannt, die einen geraden hinteren Abschnitt aufweist, der mit einer Biegung in einen etwa halbkreisförmigen vorderen Abschnitt übergeht, wobei die Spitze der Nadel auf der Höhe der Verlängerung des geraden hinteren Abschnitts liegt. Hierbei sind verschiedene Querschnittsformen der Nadel vorgesehen.

Durch die Fortschritte der Endoskopie ist es möglich geworden, intrakorporal Nähte zu legen. Das dafür zur Verfügung stehende Instrumentarium und Nahtmaterial ist dafür aber nur bedingt geeignet. Die geraden Nadeln lassen sich wegen fehlender Bewegungsfreiheit nur schwer dirigieren und erschweren die Verbindung von Gewebeteilen. Kreisbogenförmige Nadeln sind entweder bei gegebenem Trokardurchmesser zu klein oder, einmal in die Bauchhöhle eingeführt, schwer in die richtige Richtung zu bringen. Der Faden neigt dazu, sich rechtwinkelig abzuknicken. Die gebräuchlichen chirurgischen Nähfäden aus geflochtenem, resorbierbarem Material sind weich und fallen in jede Richtung. Es ist deshalb schwierig, das Fadenende für die Knotenbildung aufzufinden, in die richtige Richtung zu lenken und zu verarbeiten.

Der Erfindung liegt die Aufgabe zugrunde, eine chirurgische Nadel so auszubilden, daß ein schnelles und sicheres Nähen in der Bauchhöhle möglich ist.

Diese Aufgabe wird erfindungsgemäß durch die Merkmale im kennzeichnenden Teil der Ansprüche 1 und 2 gelöst.

Durch diese Formgebung kann die Nadel durch mikrochirurgische Instrumente besser gehalten und geführt werden, so daß das Nähen in der Bauchhöhle vereinfacht wird.

Vorteilhafte Ausgestaltungen der Erfindung sind in der nachfolgenden Beschreibung und in den weiteren Ansprüchen angegeben.

Die Erfindung wird beispielsweise anhand der Zeichnungen näher erläutert. Es zeigen
- Fig. 1: schematisch eine Knotenbildung mittels Nadel und Faßzange,
- Fig. 2: ebenfalls eine Knotenbildung bei einer anderen Nahtführung,
- Fig. 3: eine Seitenansicht einer erfindungsgemäß geformten Nadel,
- Fig. 4: eine weitere Ausführungsform der erfindungsgemäß geformten Nadel, und

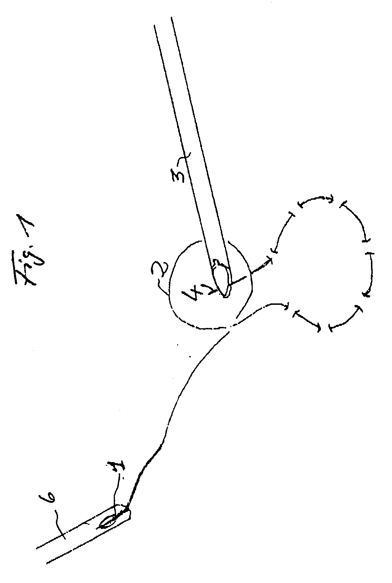

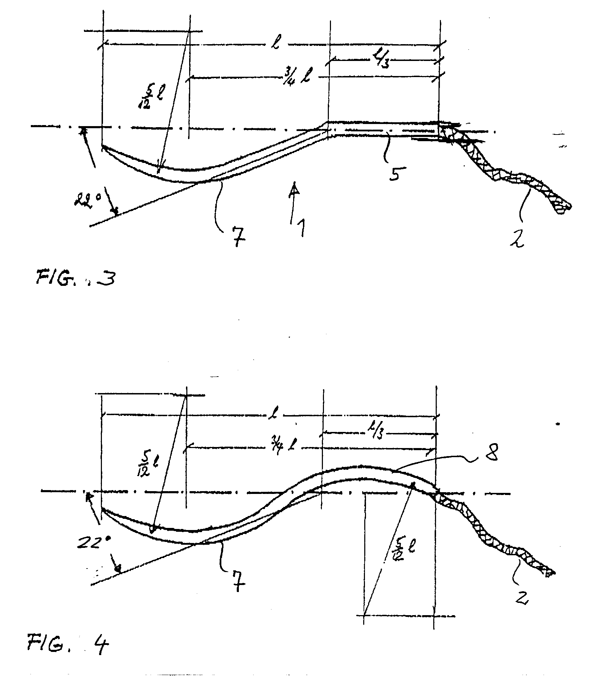

Fig. 1 zeigt bei 1 in einer Draufsicht eine Nadel, die, wie Fig. 3 zeigt, in der Seitenansicht löffelförmig gebogen ist. Mit 2 ist ein an der Nadel 1 befestigter Nähfaden bezeichnet, der zumindest abschnittsweise versteift ist. Mit 3 ist eine an sich bekannte Faßzange bezeichnet, die das Fadenende 4 hält, um dieses durch die etwa kreisförmig wiedergegebene Fadenschleife zur Knotenbildung zu ziehen, nachdem mittels der Nadel 1 eine tabakbeutelförmige Naht durch das Gewebe gezogen ist, wie durch Unterbrechungen des Fadens 2 in Fig. 1 dargestellt. Mit 6 ist in Fig. 1 ein pinzettenartiges Haltegerät bezeichnet, mittels dem die Nadel 1 gehalten und geführt wird.

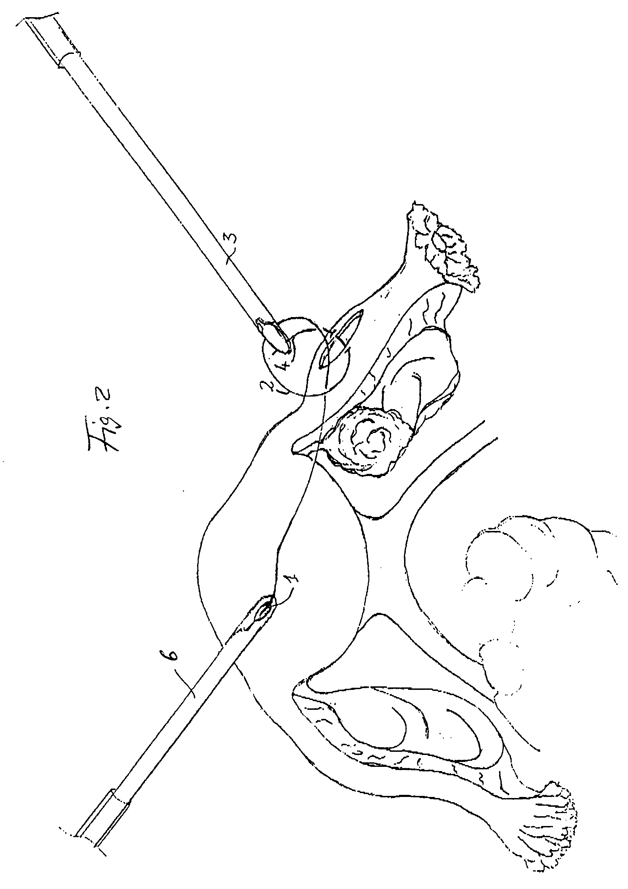

Fig. 2 zeigt in ähnlicher Darstellung die Knotenbildung bei einer anderen Nahtführung. Auch in diesem Falle wird mittels der Faßzange 3 das Fadenenede 4 durch eine Schlinge des Fadens 2 gezogen.

Wie Fig. 2 zeigt ist die Nadel 1 in der Draufsicht gerade. Wie Fig. 3 zeigt, ist die Nadel 1 nach einer ersten Ausführungsform in der Seitenansicht in der Weise gebogen, daß der sich an die Nadelspitze anschließende Abschnitt 7 flach teilkreisförmig nach unten gebogen ist und sich in Richtung des Abschnitts 7 ein gerader Endabschnitt 5 anschließt, an dessen Ende der Nähfaden 2 befestigt ist. Die Nadel 1 hat somit etwa die Form eines Löffels in der Seitenansicht.

Bei der Ausführungsform nach Fig. 3 ist an dem geraden Endabschnitt 5 der Nadel 1 der Faden 2 in einer Längsbohrung der Nadel eingeklemmt. Es ist aber auch möglich, den Faden 2 in einer Öse am hinteren Nadelende zu befestigen.

Bei diesem Ausführungsbeispiel geht der gerade Abschnitt 5 in eine Schräge unter einem Winkel von etwa 22° nach unten über, wobei die Nadel nach einem Abschnitt von 3/4 1 mit einem Radius von 5/12 1 nach oben gebogen ist. Die Nadelspitze liegt dabei unter der Geraden, welche die Fortsetzung des geraden Abschnitts 5 bildet. Der gerade Abschnitt 5 hat eine Länge von 1/3, wobei 1 die Gesamtlänge der Nadel 1 ist.

Fig. 4 zeigt eine abgewandelte Ausführungsform der Nadel, wobei der hintere Endabschnitt der Nadel bei 8 nach oben bogenförmig gekrümmt ist, so daß sich insgesamt etwa eine flache S-Form der Nadel in der Seitenansicht ergibt. Die flache Krümmung des hinteren Abschnitts 8 entspricht im wesentlichen der gegenläufigen Krümmung des vorderen Abschnitts 7. Die in Fig. 4 angegebenen Maße entsprechen im wesentlichen denen in Fig. 3.

Der Nähfaden, bei dem es sich insbesondere um einen resorbierbaren Faden handelt, ist zumindest abschnittsweise im Nähbereich so präpariert, daß er während des Nähvorganges steif bleibt und eine drahtartige Konsistenz hat, so daß er steif, elastisch und biegsam ist. Diese Eigenschaft beeinflußt die Resorbierbarkeit nicht. Der Faden wird dazu bei der Herstellung beispielsweise mit Acrylat, Wachs oder Saccharose präpariert, so daß er die gewünschte Konsistenz erhält. Beim Nähen läßt sich ein solcher Faden sowohl leicht dirigieren als auch durch einfaches Gegeneinanderschieben zu einer Schleife formen, so daß das Knotenschlingen erheblich erleichtert und beschleunigt wird.

Das Fadenende 4 mit dem vorkonfektionierten Knoten wird auf einem kurzen Abschnitt mit magnetisierbarem, physiologisch unbedenklichem Material präpariert. Mit einer magnetischen Faßzange 3 läßt sich dadurch das Fadenende 4 jederzeit leicht auch aus versteckten Winkeln auffinden und zur Knotenbildung durch die bereits geformte Schlinge ziehen. Das Fadenende 4 kann beispielsweise mit Metallstaub mittels eines Klebers beschichtet werden, wobei durch diese Metallstaubbeschichtung das Fadenende durch eine magnetische Faßzange angezogen wird.

Es ist auch möglich, Magnetteilchen am Fadenende mittels eines Klebemittels aufzubringen. Das auf diese Weise präparierte Fadenende kann nach der Knotenbildung abgetrennt und entfernt werden.

Die Kombination aus flach gekrümmter Nadel im vorderen Abschnitt mit versteiftem, am Endabschnitt magnetisierbarem Nähfaden stellt eine wesentliche Verbesserung für die intrakorporale Nahttechnik mittels Mikrooperationsinstrumenten dar, wobei das Nähen schnell und sicher durchgeführt werden kann.

Die Nadel kann am vorderen Abschnitt 7 einen kreisförmigen, ovalen oder auch dreieckigen Querschnitt haben. Der Abschnitt 7 ist vorzugsweise teilkreisförmig gekrümmt, er kann aber auch flach elliptisch gestaltet sein.

## Patentansprüche

1. Chirurgische Nadel mit einem in Seitenansicht gekrümmten, zur Spitze hin verlaufenden vorderen Abschnitt (7), an den sich ein gerader hinterer Abschnitt (5) anschließt,
**dadurch gekennzeichnet,**
daß sich der gekrümmte vordere Abschnitt (7) aus einem geraden Teil, der in einem flachen Winkel von dem geraden hinteren Abschnitt (5) ausgeht, und einem flachgekrümmten Teil zusammensetzt, dessen Spitze unter der Verlängerungslinie des geraden hinteren Abschnitts (5) liegt, wobei der Mittelpunkt der Krümmung auf ca. drei Viertel der Länge (1) der Nadel (1) ausgehend vom hinteren Ende der Nadel liegt.

2. Chirurgische Nadel mit einem in Seitenansicht gekrümmten, zur Spitze hin verlaufenden vorderen Abschnitt (7) der Nadel, an den sich ein hinterer Abschnitt (8) der Nadel anschließt,
**dadurch gekennzeichnet,**
daß der hintere Abschnitt (8) der Nadel gegenläufig zum vorderen Abschnitt (7) der Nadel gekrümmt ist, wobei das hintere Ende des hinteren Abschnittes (8) auf der Längsachse der Nadel liegt, die durch den Übergangspunkt zwischen den gegenläufigen Krümmungen des vorderen und hinteren Abschnittes (7,8) der Nadel verläuft,
daß sich der gekrümmte vordere Abschnitt (7) aus einem mittleren Teil, der einen flachen Winkel zur genannten Längsachse der Nadel bildet, und einem vorderen flachgekrümmten Teil zusammensetzt, dessen Spitze unter der genannten Längsachse liegt, wobei der Mittelpunkt der Krümmung des vorderen flachgekrümmten Teils auf ca. drei Viertel der Länge (1) der Nadel (1) ausgehend vom hinteren Ende der Nadel liegt.

3. Nadel nach Anspruch 1,
dadurch gekennzeichnet,
daß der gerade Teil des gekrümmten vorderen Abschnitts (7) in einem flachen Winkel von etwa 22 ° von dem geraden hinteren Abschnitt (5) ausgeht.

4. Nadel nach den Ansprüchen 1 bis 3,
dadurch gekennzeichnet,
daß die Krümmung des vorderen Abschnitts einen Radius von ca. 5/12 der Länge (1) der Nadel hat.

5. Nadel nach Anspruch 2,
dadurch gekennzeichnet,
daß der Radius der Krümmung des hinteren Abschnitts (8) dem der Krümmung des vorderen Abschnitts (7) entspricht.

6. Nadel nach den vorhergehenden Ansprüchen,
dadurch gekennzeichnet,
daß ein Nähfaden (2) mit dem hinteren Ende der Nadel (1) verpresst ist.

7. Nadel nach den vorhergehenden Ansprüchen,
dadurch gekennzeichnet, daß die Spitze der Nadel einen kreisförmigen Querschnitt aufweist.

8. Nadel nach den Ansprüchen 1 bis 7,
dadurch gekennzeichnet,
daß die Spitze der Nadel einen etwa dreieckigen Querschnitt aufweist.

## Claims

1. Surgical needle comprising a front portion (7) curved in lateral view and extending to the point of the needle, to which portion (7) a straight rear portion (5) is connected,
**characterized in that**
the curved front portion (7) starts from a straight portion, which starts at a flat angle from the straight rear portion (5), and a flat-curved portion, the point of which is under the elongation line of the straight rear portion (5), wherein the center of the curvature is on about three quarters of the length (1) of the needle (1) starting from the rear end of the needle.

2. Surgical needle comprising a front portion (7) of the needle, which portion (7) is curved in lateral view and is extending to the point of the needle, and to which portion (7) a rear portion (8) of the needle is connected,
characterized in that
the rear portion (8) of the needle is curved in opposite direction of the front portion (7) of the needle, wherein the rear end of the rear portion (8) is on the longitudinal axis of the needle, which extends through the transition point between the curves of the front and rear portion (7,8) of the needle, which curves move in opposite directions,
that the curved front portion (7) consists of a middle portion which forms a flat angle to the mentioned longitudinal axis of the needle, and a front flat-curved portion, the point of which is under the mentioned longitudinal axis, wherein the center of the curvature of the front flat-curved portion is on about three quarters of the length (1) of the needle (1) starting from the rear end of the needle.

3. Needle according to claim 1,
characterized in that
the straight portion of the curved front portion (7) starts at a flat angle of about 22° from the straight rear portion (5).

4. Needle according to claims 1 to 3,
characterized in that
the curvature of the front portion has a radius of about 5/12 of the length (1) of the needle.

5. Needle according to claim 2,
characterized in that
the radius of the curvature of the rear portion (8) corresponds to the radius of the curvature of the front portion (7).

6. Needle according to the preceding claims,
characterized in that
a sewing thread (2) is pressed with the rear end of the needle (1).

7. Needle according to the preceding claims,
characterized in that
the top of the needle has a circular cross-section.

8. Needle according to claims 1 to 7,
characterized in that
the point of the needle has an approximately three-angular cross-section.

## Revendications

1. Aiguille chirurgicale comportant une partie antérieure (7) courbée en vue latérale et s'étendant à la pointe, a quelle partie (7) une partie droite derrière (5) est connectée,
**caractérisée en ce que**
la partie courbée antérieure (7) est composée d'une partie droite qui part à an angle plat de la partie droite derrière (5), et une partie courbée platement, la pointe de laquelle est dessous de la ligne d'allongement de la partie droite derrière (5), la centre de la courbure étant à approximativement trois quarts de la longueur (1) de l'aiguille (1) partant du bout derrière de l'aiguille.

2. Aiguille chirurgicale comportant une partie antérieure (7) courbée en vue latérale et s'étendant à la pointe, a quelle partie une partie derrière (8) de l'aiguille est connectée,
caractérisée en ce que
la partie derrière (8) de l'aiguille est courbée marchant en sens contraire à la partie antérieure (7) de l'aiguille, le bout derrière de la partie derrière (8) étant sur l'axe longitudinal de l'aiguille, qui s'étend par la pointe de transition entre les courbures de la partie antérieure et derrière (7,8) de l'aiguille, quelles courbures marchent en sens contraire,
que la partie courbée antérieure (7) est composée d'une partie central, qui forme un angle plat avec l'axe longitudinal de l'aiguille, et une partie courbée platement antérieure, la pointe de laquelle est sous ledit axe longitudinal, la centre de la courbure de la partie courbée platement antérieure étant positionnée à approximativement trois quarts de la longitude (1) de l'aiguille (1) partant du bout derrière de l'aiguille.

3. Aiguille selon la revendication 1,
caractérisée en ce que
la partie droite de la section courbée antérieure (7) part en un angle plat d' approximativement 22° de la partie droite derrière (5).

4. Aiguille selon les revendications 1 à 3,
caractérisée en ce que
la courbure de la partie antérieure a un rayon d'approximativement 5/12 de la longitude (1) de l'aiguille.

5. Aiguille selon la revendication 2,
caractérisée en ce que
le rayon de la courbure de la partie derrière (8) correspond à la courbure de la partie (7) antérieure.

6. Aiguille selon les revendications précédentes,
caractérisée en ce que
un fil à coudre (2) est comprimé avec le bout derrière de l'aiguille (1).

7. Aiguille selon les revendications précédentes ,
caractérisée en ce que
la pointe de l'aiguille a une section transversale circulaire.

8. Aiguille selon les revendications 1 à 7,
caracterisée en ce que
la pointe de l'aiguille a une section transversale approximativement triangulaire.
